# DEMANDE DE BREVET EUROPEEN

(11) **EP 4 717 208 A1**
(43) Date de publication de la demande: **01.04.2026**
(21) Numéro de dépôt: 24202388.5
(22) Date de dépôt: 25.09.2024
(51) Int. Cl.: A61B 17/88, A61F 2/46, A61F 2/30

(54) **IMPLANT OSSEUX EXPANSIBLE DE CHIRURGIE ORTHOPÉDIQUE ET SYSTÈME ORTHOPÉDIQUE**

(71) Demandeur: Lock-In VCF SA, 1180 Rolle (CH)
(72) Inventeur: LACAZE, Guillaume, 1278 La Rippe (CH)
(74) Mandataire: Gsmart

(57) **Abrégé**

La présente invention concerne un Implant osseux expansible de chirurgie orthopédique humaine, un Système orthopédique et un Procédé de fabrication de l'implant, pour la restauration de volume et/ou géométrie d'un os, par une expansion entre une configuration repliée et une configuration déployée, ledit implant comportant au moins deux faces comprenant chacune au moins un plateau (13, 14, 15) pour le contact avec les tissus osseux, chacun des plateaux étant supportés par au moins deux bras de support (131, 141, 151) chacun, par l'intermédiaire d'une charnière sur l'axe central (3) et d'une charnière sous le plateau (13, 14, 15) respectif de chacun desdits bras de support (131, 141, 151), caractérisé en ce que :
- une douille ou bague d'expansion (20) disposée dans le même axe que ledit axe central (3) ;
- au moins deux bras d'expansion (132, 142, 152) comportent chacun une charnière les reliant à l'une des extrémités de l'un des plateaux (13, 14, 15) et une charnière les reliant à ladite douille ou bague d'expansion (20) ;
- ladite douille ou bague d'expansion (20) et l'extrémité proximale de l'axe central (3) sont aptes à coopérer, respectivement ou inversement, avec un tube creux (A1) de préhension de l'implant (1) d'un instrument d'implantation (A) et avec une tige d'expansion (A3) dudit instrument (A) ;
-ladite tige d'expansion (A3) est apte à coulisser à l'intérieur dudit tube creux (A1), de façon à permettre de provoquer un éloignement entre ladite douille (3) et ledit axe central (3), exerçant une traction sur les plateaux (13, 14, 15), par l'intermédiaire des bras d'expansion (132, 142, 152), ce qui engendre un pivotement desdits bras de support (131, 141, 151) provoquant l'éloignement des plateaux (13, 14, 15) par rapport à l'axe central (3), de manière à résulter en une expansion contrôlée de l'implant (1) entre ladite configuration repliée et ladite configuration déployée.

## Description

La présente demande se rapporte au domaine de la chirurgie, en particulier la chirurgie orthopédique humaine et notamment du traitement d'une structure osseuse affaissée, par une restauration du volume (ou redressement) de cette structure osseuse. La présente demande concerne en particulier un implant et son procédé de fabrication, ainsi qu'un système pour la restauration de structure osseuse, notamment dans le rachis pour le traitement (souvent appelé « réduction ») des fractures de compression, notamment vertébrales (VCF pour l'anglais « Vertébral Compression Fracture »).

Dans ce domaine, le problème de la restauration du volume de structure osseuse qui s'est affaissée est bien connu et la littérature abonde de solutions utilisant des implants expansibles capables de passer d'une configuration repliée à une configuration déployée pour restaurer la hauteur de la structure osseuse, de préférence en combinaison avec une injection de ciment de substitution osseuse, dit ciment (ou ciment osseux). De nombreux ciments sont connus et ils présentent tous l'avantage d'être injectables à l'état liquide ou visqueux pendant une certaine durée, puis de durcir (par polymérisation) à l'intérieur de la structure osseuse pour la stabiliser.

Un problème majeur dans ce domaine concerne l'expansion de l'implant pour restaurer une hauteur au tissu osseux lésé. De nombreuses solutions sont connues de l'art antérieur, comme notamment les demandes de brevet EP3086729, US11540926, EP3747385, EP2572680, EP3958752, EP2693967, EP2405835, US9579130, EP4216836, WO2023122005, WO2022162418, EP3843668 ou US10945861 mais ces solutions présentent divers problèmes de difficulté de manipulation pour le déploiement, de stabilité et de fiabilité une fois déployés. De plus, ces solutions connues s'accompagnent généralement d'une injection de ciment osseux mais ne fournissent aucun enseignement relatif à la fuite de ciment en dehors de l'implant, alors qu'une telle fuite peut être préjudiciable pour les tissus environnants, voire même l'organisme tout entier si les substances chimiques du ciment envahissent le système sanguin. En effet, le ciment comporte en général une ou plusieurs substances chimiques polymérisables, par exemple comme le poly(méthacrylate de méthyle) (PMMA, pour l'anglais poly-methyl-methacrylate) et éventuellement des additifs. De plus, la température atteinte lors de la polymérisation du ciment n'est pas inoffensive car elle est généralement supérieure à 60°.

Il est connu de l'art antérieur, notamment des document EP1308134, US9510877, US8936627 ou EP2467099, des dispositifs de redressement et stabilisation (ou réduction de fracture d'os), notamment du rachis sous la forme de stent, ou sous forme de ballons ou de sacs gonflables poreux comme dans les documents EP1408888 ou EP1379185, éventuellement équipé de plateaux de support comme dans le document US20060100706. De nombreux documents proposent ce genre de stent, c'est-à-dire d'endoprothèse déformable similaire aux endoprothèses, extenseurs ou tuteurs vasculaires, qui sont généralement sous la forme d'un corps tubulaire maillé, le plus souvent métallique et déformable par l'introduction d'un ballon gonflable pour dilater le corps en écartant les mailles, le ballon étant ensuite retiré pour permettre une injection de ciment, qui durcit et forme ainsi une structure de redressement et de stabilisation. Cependant, ces dispositifs présentent l'inconvénient de nécessiter une implantation en deux temps avec le gonflage du ballon puis l'injection de ciment, ce qui ralentit et complexifie l'opération mais présente également un risque d'affaissement du dispositif entre le dégonflage du ballon et le remplissage du stent par le ciment. D'autre part, ces solutions présentent l'inconvénient de ne pas répondre au problème majeur des fuites de ciment.

Il est également connu, notamment des documents EP1938765, EP2351539 ou WO200434924, des solutions utilisant des implants à structure maillée, en métal à mémoire de forme, qui est contrainte dans une forme repliée pour l'insertion dans le tissu osseux et capable de s'expandre spontanément, lors du relâchement de la contrainte et/ou sous l'effet de la chaleur. Ces solutions présentent l'inconvénient d'une utilisation d'alliages coûteux et une fabrication complexe pour obtenir une mémoire de forme adéquate qui convienne à l'implantation visée, ce qui implique un surcoût en multipliant le nombre d'implants différents nécessaires pour couvrir les différents cas pathologiques, notamment par l'amplitude de la déformation dont est capable le matériau à mémoire de forme. De plus, la force exercée par le retour du métal à sa forme non contrainte n'est souvent pas suffisante pour redresser correctement la structure osseuse qui s'est affaissée, ou du moins limitative. D'autre part, ces solutions présentent également l'inconvénient de ne pas répondre au problème majeur des fuites de ciment.

Il est également connu de l'art antérieur, notamment des documents EP2405835, US9579130, EP2572680 ou EP1956990 des solutions utilisant des implants expansibles utilisant un mécanisme de levier, à la manière d'un cric de voiture (« jack » en anglais) pour restaurer la structure osseuse à une hauteur déterminée. Ces solutions présentent l'avantage de ne pas risquer d'affaissement contrairement à un sent déployés par un ballon qui est retiré avant l'injection de ciment, mais ils présentent également l'inconvénient d'une implantation en deux temps et que les dimensions de la surface de support pour exercer la force d'expansion sur le tissu osseux est limitée, en comparaison avec les stents notamment. D'autre part, ils présentent également les inconvénients d'être coûteux et de ne pas répondre non plus au problème majeur des fuites de ciment.

Le problème de fuite de ciment a déjà été identifié, notamment dans les documents EP1408888, EP1509175 ou WO200394805 qui expriment l'intérêt que présenterait un implant déformable peu perméable ou imperméable pour limiter ou éviter les fuites de ciment. Ces documents envisagent de nombreuses solutions pour un implant expansible, en métal ou en polymère, qui pourrait être soit souple et flexible comme une membrane ou un tissu, voire élastique, soit semi-rigide (« conformable ») ou rigide, soit en matériau à mémoire de forme, avec une paroi continue ou fenestrée (i.e., maillée) et qui pourrait être poreux ou non poreux. Cependant, toutes ces hypothèses décrites dans ces documents définissent surtout des méthodes envisageables de traitement et des objectifs à atteindre, sans fournir de réel enseignement en ce qui concerne les caractéristiques techniques, ou l'agencement structurel des implants, ni sur la façon d'obtenir de tels implants et ainsi mettre en oeuvre ces méthodes. Ces propositions présentent donc un problème majeur de faisabilité technique.

D'autre part, un problème qui n'est pas identifié dans l'art antérieur concerne du site d'injection du ciment et la répartition des forces exercées sur les tissus osseux pour les redresser. En effet, l'imperméabilité d'un implant permet d'éviter les fuites de ciment, mais la nature de la membrane imperméable et ses caractéristiques techniques comme ses propriétés physico-chimiques et mécaniques influent sur sa capacité à se déployer sans se rompre et à redresser la structure osseuse. Ainsi, une membrane élastique présente l'inconvénient de se déformer excessivement dans les zones à faible densité et d'avoir ainsi une capacité limitée à restaurer une hauteur, avec en plus le risque de se rompre aux endroits où son élasticité maximale est dépassée à cause de cette déformation incontrôlée. Une membrane semi-rigide est donc préférable, mais ce problème de déformation implique également un problème de formes de l'implant, en configuration repliée et surtout en configuration déployée. En effet, la forme de l'implant déployé délimite le site d'injection du ciment et la maîtrise de ce site est importante pour la répartition des forces conduisant à remplir les zones à faible densité tout en redressant la structure (notamment en hauteur), ce qui impacte la réussite de l'opération. Ainsi, on comprend que la fourniture d'un implant répondant à l'ensemble de ces problèmes s'accompagne d'un problème de faisabilité technique et donc de fabrication.

D'autres problèmes récurrents en chirurgie orthopédique concernent l'invasivité (c'est-à-dire le but de faire une incision et des lésions les moins étendues possibles) mais également le ratio de déploiement afin d'obtenir un implant déployé qui comble le plus grand volume possible tout en ayant été introduit par un passage le plus petit possible. D'autre part, ce ratio de déploiement va impacter la répartition des forces pour redresser les vertèbres : si on est trop déformable, la pression d'injection du ciment va plutôt déformer la poche au lieu de restaurer la hauteur.

Un problème complémentaire à celui du déploiement concerne le repliement qui n'est généralement pas possible dans les implants de l'art antérieur. La maîtrise du repliement permet la maîtrise du déploiement et donc du site d'injection avec une répartition homogène du ciment et de la pression pour remplir l'espace à combler suite à l'affaissement. Homothétie parfaite s'adaptant à la fracture en respectant la forme de l'os à l'intérieur de la fracture.

Dans ce contexte, on comprend qu'il persiste dans le domaine un problème technique concernant la restauration de structure osseuse (redressement ou réduction de fracture ou augmentation de volume après affaissement) grâce à un implant expansible (déployable) qui soit capable d'expandre les tissus osseux affaissés et suffisamment imperméable pour éviter ou limiter la fuite de ciment en-dehors de l'implant avec une maîtrise du site d'injection.

Enfin, un problème principal qui persiste dans le domaine concerne la faisabilité technique de la fabrication des implants proposés dans l'art antérieur. Il est par exemple connu le document WO200394805 décrit de nombreuses méthodes d'administration de substances et notamment de ciment osseux, avec de nombreuses variantes envisagées pour un implant expansible, en métal ou en polymère, qui pourrait être soit souple et flexible comme une membrane ou un tissu, soit semi-conformable ou rigide, soit en matériau à mémoire de forme, avec une paroi continue ou fenestrée (i.e., maillée) et qui pourrait être poreux ou non poreux. Cependant, ce document ne décrit que des méthodes envisageables de traitement, mais ne fournit aucun enseignement en ce qui concerne les caractéristiques techniques, ou l'agencement structurel de ces nombreux implants hypothétiques utilisés pour ces méthodes envisagées, ni sur la façon d'obtenir de tels implants et ainsi réellement mettre en oeuvre ces méthodes. Ces propositions présentent donc un problème majeur de faisabilité technique et définissent plus des buts à atteindre que des moyens d'y parvenir. De plus, même si de nombreux objectifs ont été détaillés dans la littérature, de nombreux implants proposés pour atteindre ces objectifs n'ont jamais vu le jour à cause de problèmes de fabrication. Pour répondre au problème de fabrication, il est nécessaire de prendre en compte les problèmes liés à au souhait de compacter / replier un « sac » (ballon/poche) en matériau rigide et imperméable pour :
- passer dans un conduit cylindrique ;
- rendre possible l'expansion sans rupture de la poche, malgré la rigidité et la différence de volume souhaité entre le volume replié et le volume déployé ;
- Maîtriser le volume et la répartition des forces d'expansion sur l'os.

Dans ce contexte, un but de la présente invention est de pallier au moins certains inconvénients de l'art antérieur en proposant un implant de restauration de structure osseuses affaissées fiable et simple à manipuler et implanter.

Ce but est atteint par un implant osseux expansible de chirurgie orthopédique humaine pour la restauration de volume et/ou géométrie d'un os, par une expansion entre une configuration repliée et une configuration déployée, ledit implant comportant un axe central et s'étendant selon un axe longitudinal entre une extrémité proximale apte à coopérer avec un instrument d'implantation pour tenir l'implant et une extrémité distale destinée à être insérée en premier dans l'os, au moins deux faces, par exemple supérieure et inférieure, de l'implant comportant chacune au moins un plateau pour le contact avec les tissus osseux, chacun des plateaux étant supportés par au moins deux bras de support chacun, par l'intermédiaire d'une charnière sur l'axe central et d'une charnière sous le plateau respectif de chacun desdits bras de support ;
caractérisé en ce que :
- une douille ou bague d'expansion disposée dans le même axe que ledit axe central ;
- au moins deux bras d'expansion comportent chacun une charnière les reliant à l'une des extrémités de l'un des plateaux et une charnière les reliant à ladite douille ou bague d'expansion ;
- ladite douille ou bague d'expansion et l'extrémité proximale de l'axe central sont aptes à coopérer, respectivement ou inversement, avec un tube creux de préhension de l'implant d'un instrument d'implantation et avec une tige d'expansion dudit instrument ;
- ladite tige d'expansion est apte à coulisser à l'intérieur dudit tube creux , de façon à permettre de provoquer un éloignement entre ladite douille et ledit axe central, exerçant une traction sur les plateaux , par l'intermédiaire des bras d'expansion , ce qui engendre un pivotement desdits bras de support provoquant l'éloignement des plateaux par rapport à l'axe central, de manière à résulter en une expansion contrôlée de l'implant entre ladite configuration repliée et ladite configuration déployée.

Selon une autre particularité, c'est ladite bague d'expansion qui est apte à coopérer avec un tube creux de préhension de l'implant dudit instrument d'implantation , en laissant passer la tige d'expansion à travers la bague, tandis que l'axe central est apte à coopérer avec ladite tige d'expansion dudit instrument, de sorte qu'une poussée exercée sur ladite tige d'expansion coulissant à l'intérieur dudit tube creux provoque l'éloignement dudit axe central par rapport à ladite bague , résultant en une expansion de l'implant contrôlée en fonction de la force de poussée exercée sur la tige d'expansion .

Selon une autre particularité, c'est l'extrémité proximale de l'axe central qui est apte à coopérer avec ledit tube creux de préhension de l'implant dudit instrument d'implantation , tandis que ladite douille ou bague d'expansion est apte à coopérer avec ladite tige d'expansion dudit instrument, de sorte qu'une poussée exercée sur ladite tige d'expansion coulissant à l'intérieur dudit tube creux provoque l'éloignement de ladite douille ou bague d'expansion par rapport audit axe central, résultant en une expansion de l'implant contrôlée en fonction de la force de poussée exercée sur la tige d'expansion .

Selon une autre particularité, l'axe central comporte un conduit apte à coopérer avec ladite tige d'expansion qui est, d'une part, creuse et munie d'au moins une ouverture au niveau de son extrémité distale et, d'autre part, connectable à un instrument d'injection de fluide pour acheminer au moins un fluide dans le site d'implantation de l'implant via l'axe central, de préférence muni d'ouvertures pour faire déboucher le fluide dans la longueur de l'axe central.

Selon une autre particularité, les bras de support des plateaux comportent deux bras reliés respectivement à proximité des extrémités proximale et distale de leur plateau respectif, pour fournir un support sur toute la longueur des plateaux et limiter leurs risques de cintrage ou fluage.

Selon une autre particularité, au moins un bras de support central supplémentaire est relié entre une portion centrale de l'axe central et une portion centrale des plateaux, avec des charnières aux deux extrémités du bras de support pour son pivotement.

Selon une autre particularité, l'implant comporte deux plateaux (13, 14) disposés de part et d'autre de l'axe central pour fournir un appui contre des tissus osseux lésés de part et d'autre de l'implant, par exemple pour restaurer une hauteur ou une largeur.

Selon une autre particularité, l'implant comporte au moins un plateau additionnel, la répartition des plateaux autour de l'axe central variant en fonction de leur nombre et/ou des besoins en termes de traitement chirurgical, avec de préférence une équi-répartition angulaire radialement par rapport à l'axe central, pour exercer une compression homogène sur les tissus osseux à la périphérie de l'implant.

Un autre but de la présente demande est de pallier au moins une partie des inconvénients de l'art antérieur en proposer un système d'intervention chirurgicale facile à utiliser et permettant une stabilisation efficace des tissus osseux

Ce but est atteint par un système de traitement orthopédique de tissu osseux lésé comprenant un ciment de substitution osseuse et au moins un instrument d'implantation et d'injection de ciment dans l'implant, caractérisé en ce qu'il comporte un implant selon l'un des modes de réalisation décrit dans la présente demande.

Selon une autre particularité, l'instrument d'implantation et d'injection de ciment comporte des moyens de contrôle de la pression et/ou de l'aspiration du ciment pour replier l'implant en configuration repliée si nécessaire.

Selon une autre particularité, l'instrument d'implantation est distinct mais complémentaire de l'instrument d'injection dont le canal d'injection du ciment traverse un canal à l'intérieur de la tige de l'instrument d'implantation tenant l'extrémité proximale de l'implant grâce à son extrémité distale.

D'autres particularités et avantages de la présente invention apparaîtront plus clairement à la lecture de la description de divers modes de réalisation ci-après, faite en référence aux dessins annexés, dans lesquels :

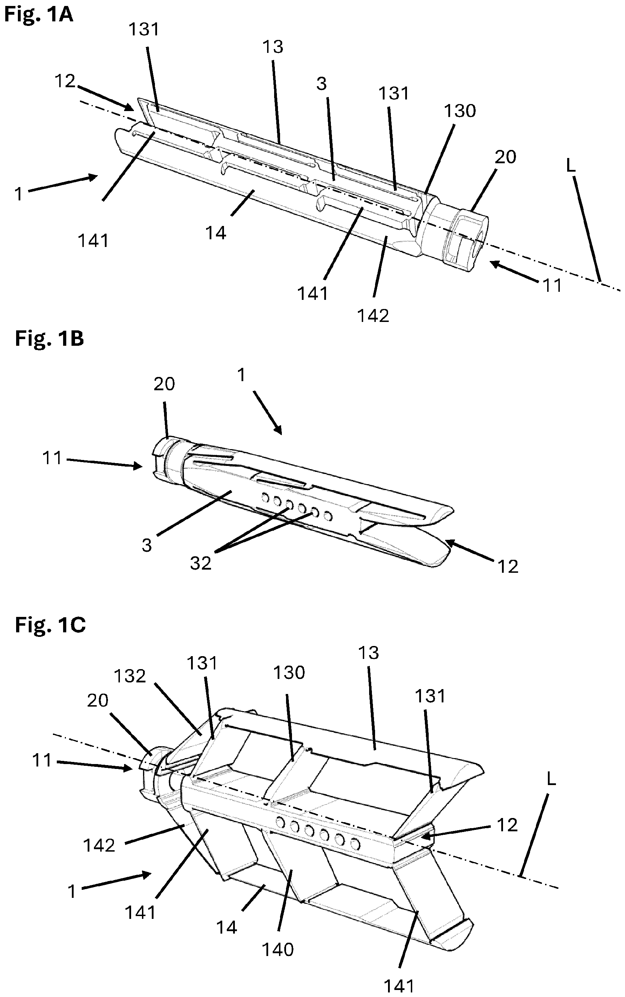

La figure 1A représente une vue en perspective d'un implant expansible selon certains modes de réalisation la figure 1B représente une vue en perspective d'un implant expansible selon un autre mode de réalisation en configuration repliée et la figure 1C représente une vie en perspective de l'implant de la figure 1B en configuration déployée ;

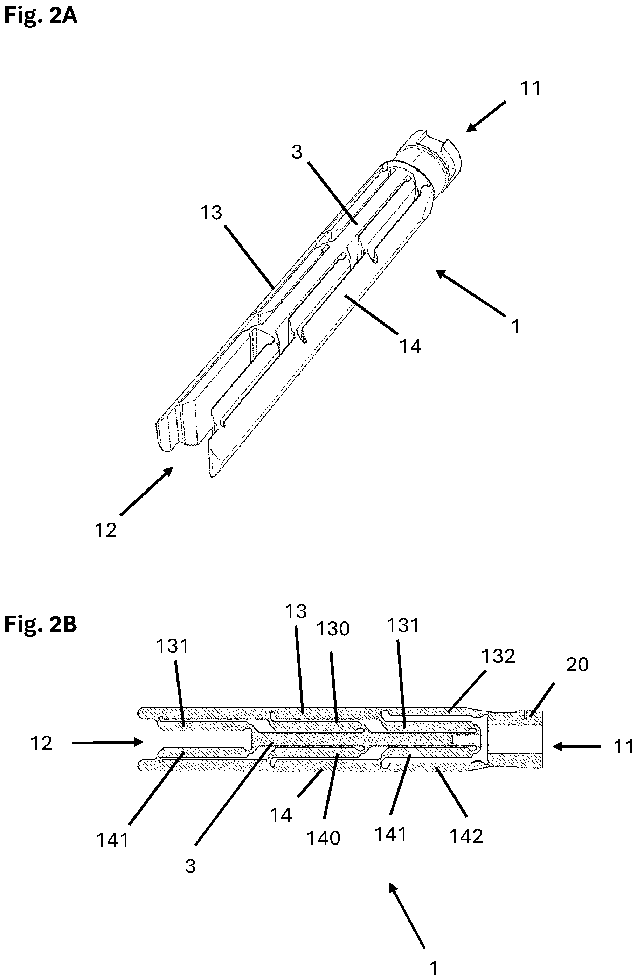

La figure 2A représente une vue en perspective d'un implant expansible en configuration repliée selon certains modes de réalisation et la figure 2B représente une vue en coupe de l'implant de la figure 1B ;
La figure 3A représente une vue de profil d'un implant expansible en configuration déployée et la figure 3B représente un détail de l'extrémité proximale d'un implant expansible selon certains modes de réalisation ;
Les figures 4A, 4B, 4C et 4D représentent des vues de profil de 4 implants expansibles en configuration rempliée selon différents modes de réalisation avec des bras de supports de différentes longueurs ;
Les figures 5A, 5B 5C et 5D représentent des vues de profil des implants respectivement des figures 4A, 4B, 4C et 4D mais en configuration déployée avec leurs plateaux non parallèles ;

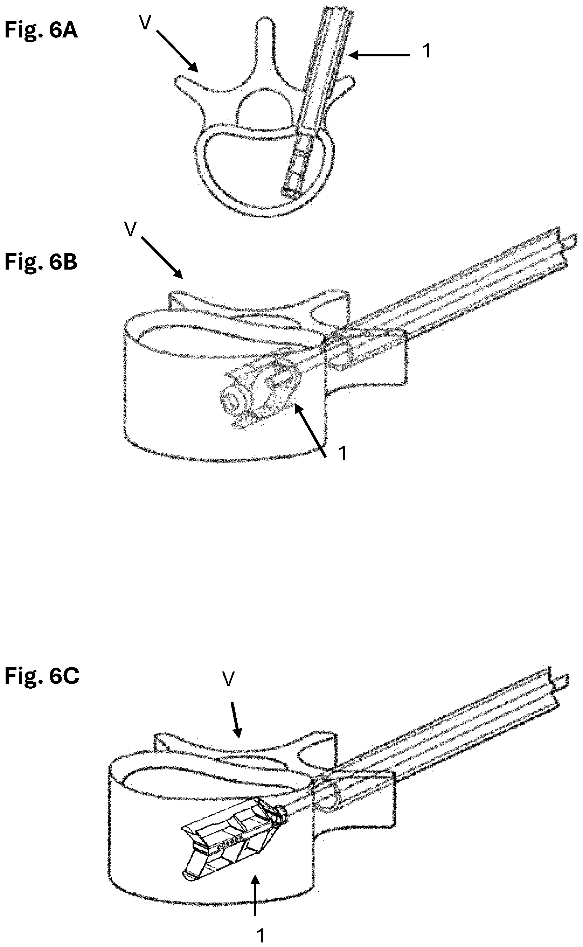

La figure 6 représente une vue de dessus d'une vertèbre dans laquelle est implanté un implant selon divers modes de réalisation ;
La figure 6B représente une vue de perspective d'une vertèbre dans laquelle est implanté un implant de l'art antérieur ;
La figure 6C représente une vue en perspective d'une vertèbre dans laquelle est implanté un implant selon certains modes de réalisation ;

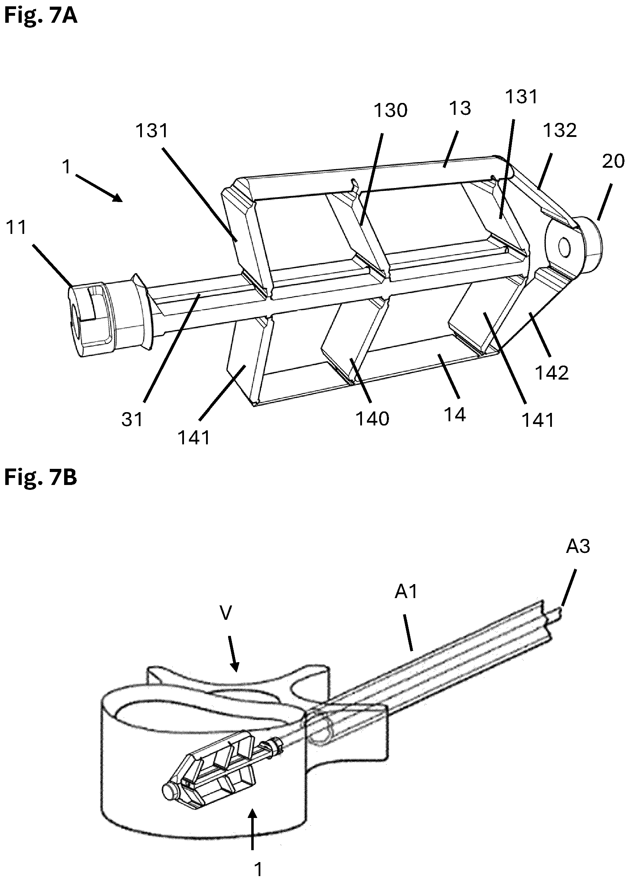

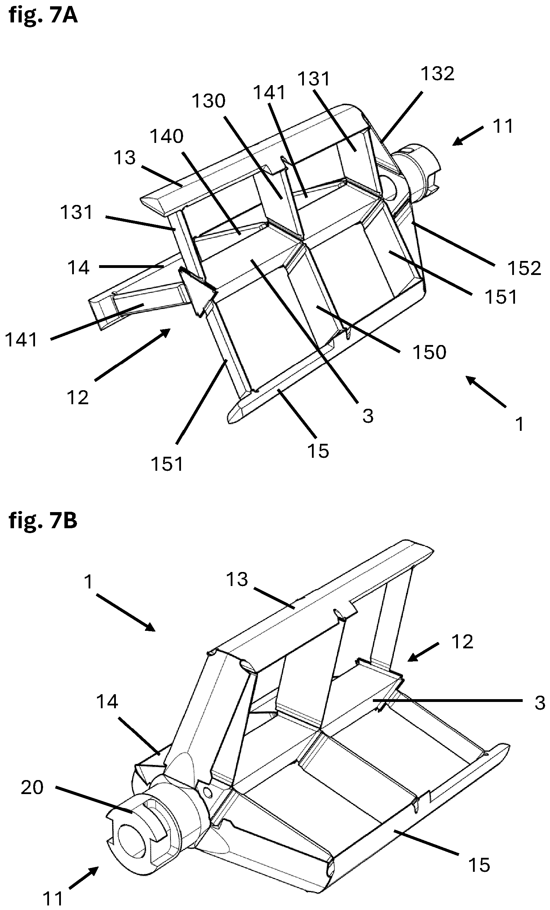

La figure 7A représente une vue en perspective d'un implant expansible selon un mode de certains de réalisation inversée et la figure 7 b représente une vue perspective d'une vertèbre dans laquelle est implanté, à l'aide d'un instrument, l'implant de la figure 7A

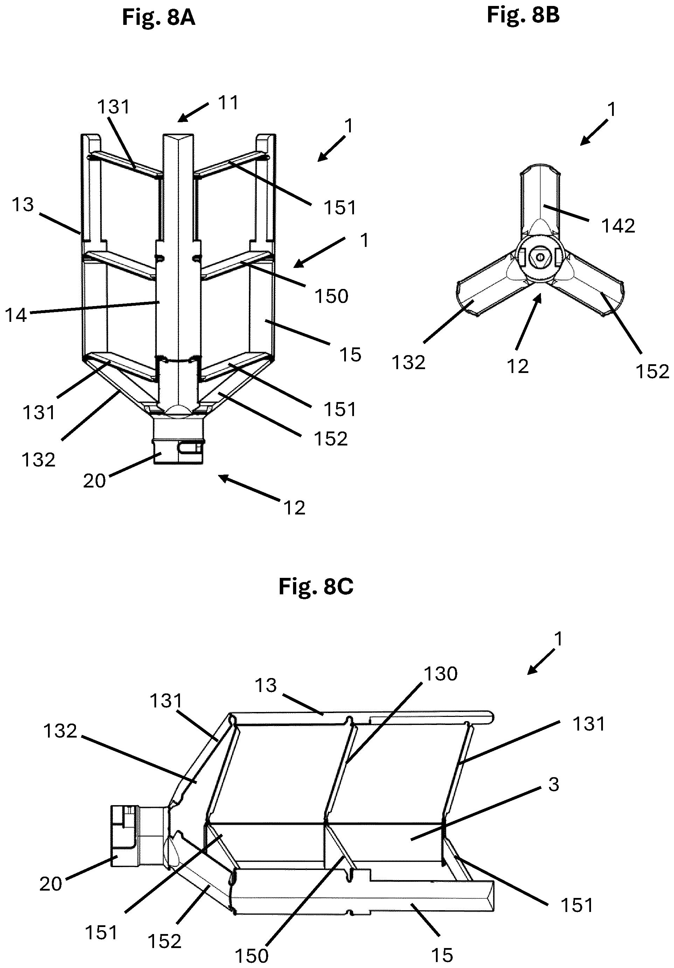

Les figures 8A, 8B et 8C représentent respectivement et une vue de dessus, une vue de face et une vue de profil d'un implant expansible à trois plateaux selon certains modes de réalisation ;
Les figures 9A et 9B représentent des vues en perspective respectivement depuis l'arrière et depuis l'avant d'un implant extensible selon les modes de réalisation des figures 8A, 8B et 8C ;

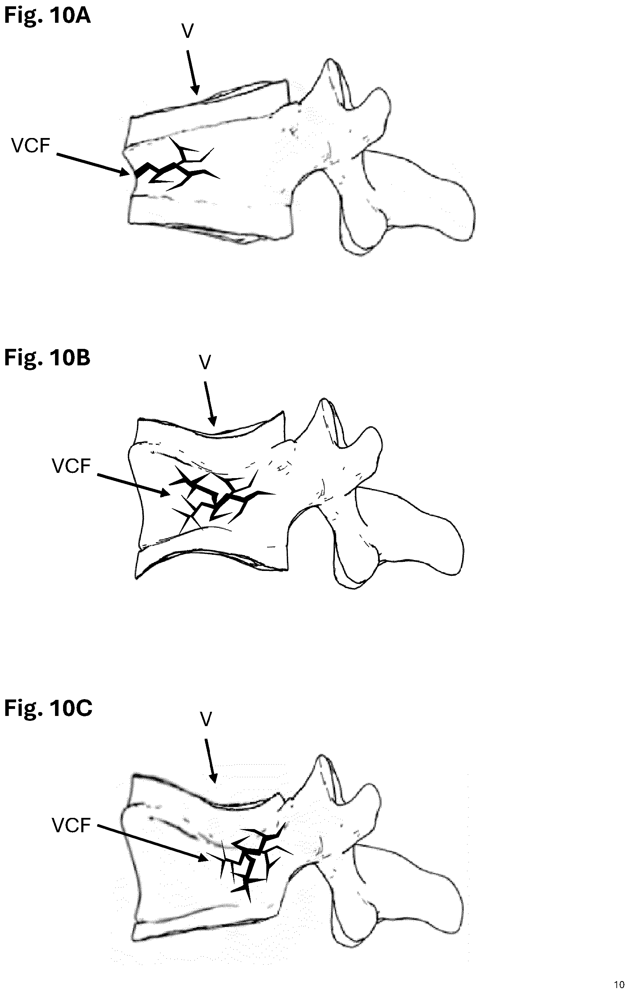

Les figures 10A, 10B et 10C représentent des vues de profil de vertèbres ayant subi des fractures de compression vertébrale (VCF) respectivement au niveau antérieur médian et postérieur ;
La figure 11A représente une vue en perspective d'un implant expansible en configuration déployée et munie d'un verrou maintenant l'implant dans sa configuration déployée, la figure 11B représente un détail de l'extrémité proximale d'un implant du type de figure 11A avec le verrou à l'extérieur de l'implant selon certains modes de réalisation, et la figure 11C représente un détail d'une de l'extrémité proximale d'un implant selon certaines réalisation avec un autre type de verrou à l'extérieur de l'implant ;
La figure 12A représente une vie en perspective d'un implant selon certains modes de réalisation et les figures 12Bet 12C représentent respectivement le détail des encarts 12B et 12C de la figure 12A de l'implant.

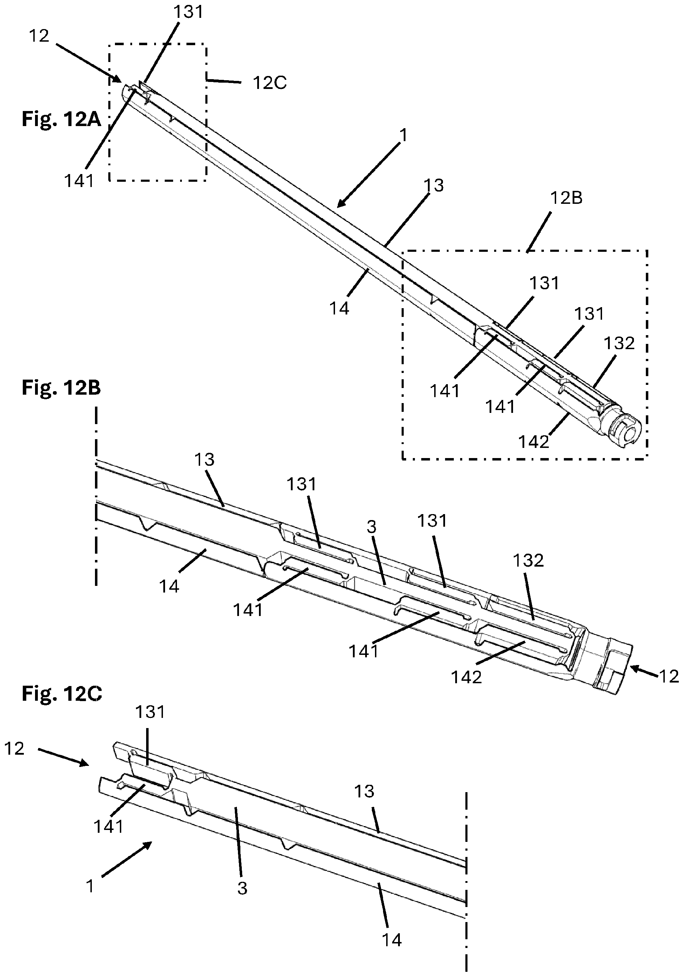

La présente demande concerne un implant et un système de chirurgie orthopédique pour le traitement d'os fracturé et de tissus osseux en général, ainsi qu'un procédé de fabrication de l'implant. L'implant osseux est de préférence un implant Rachidien, et en particulier vertébral ou même en fait intravertébral, mais d'autres utilisations sont envisageables ailleurs dans le rachis (épines intervertébrales) ou dans d'autres structures osseuses où il est nécessaire de combler un espace laissé résultant d'une fracture (dont les causes peuvent être diverses, même si elles impliquent généralement une diminution de densité osseuse). Ainsi, les fractures vertébrales de compression (VCF pour l'anglais Vertébral Compression Fracture) sont une application favorite mais ne sont pas les seules à pouvoir être traitées grâce à la présente invention et l'homme de métier appréciera les possibilités offertes sans nécessiter plus de détails ici. Comme autre os, on peut citer le fémur ou l'humérus (tête) par exemple en cas de risque d'effondrement et l'implant est implantable directement dans le canal médullaire. Certains modes de réalisation comportant plus de deux plateaux peuvent notamment servir plus efficacement au traitement d'os longs de ce type en répartissant les forces d'expansion sur plus deux surfaces, ce qui fournit une meilleure stabilité quel que soit le type d'os. Cependant, l'utilisation de l'implant dans les os longs n'est pas limitée aux modes de réalisation à plus de deux plateaux car selon le type de fractures, un implant à deux plateaux peut rester adapté à une utilisation dans les os longs, comme par exemple représenté sur les figures 12A, 12B et 12C. Les seules particularités notables pour le traitement des os longs sont la longueur des plateaux et surtout la longueur des bras support qui varie généralement en fonction de la position à l'intérieur du canal médullaire. En particulier, le canal médullaire est généralement plus large aux extrémités de l'os qu'au centre et divers modes de réalisation permettent d'épouser cette forme du canal médullaire, grâce à la présence de bras support (131, 141) de différentes longueurs pour obtenir une expansion plus ou moins importante à différents endroits de l'implant, comme par exemple représenté sur les figures 12B et 12C. Ainsi, avec des bras support (131, 141) plus long aux extrémités, l'expansion de l'implant sera plus importante aux extrémités de l'implant, qui seront donc disposées au niveau des épiphyses de l'os long par exemple. On notera que dans ce genre de cas, on peut prévoir une articulation des plateaux eux-mêmes dans les zones dans lesquelles l'expansion est variable. Une telle articulation peut naturellement consister en un amincissement des plateaux aux endroits adaptés en fonction des longueurs des bras support, par exemple comme représenté sur les figures 12B et 12C.

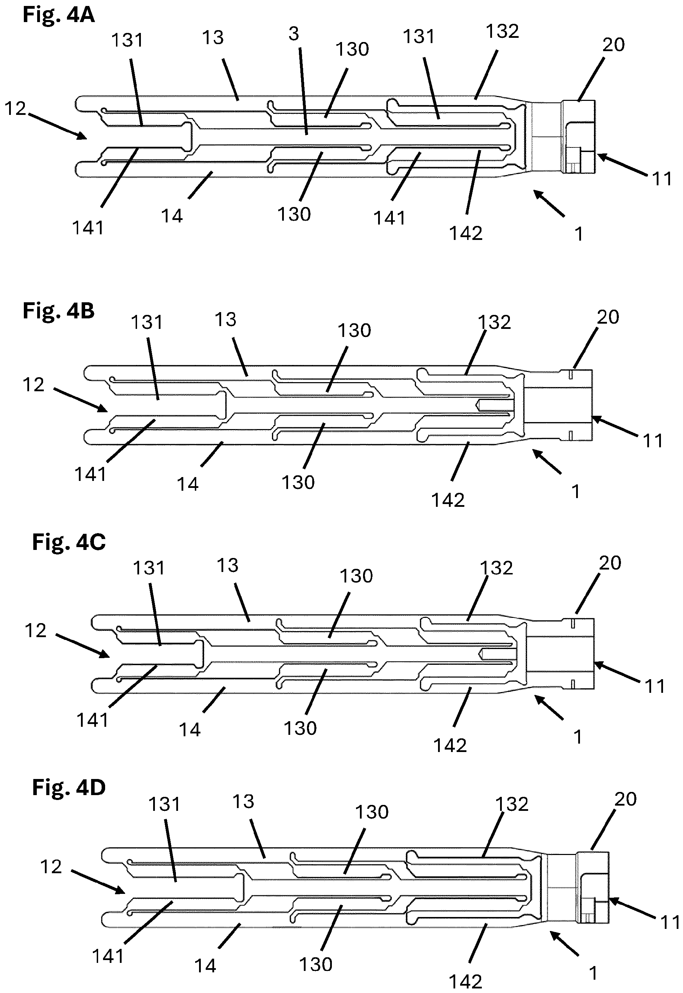

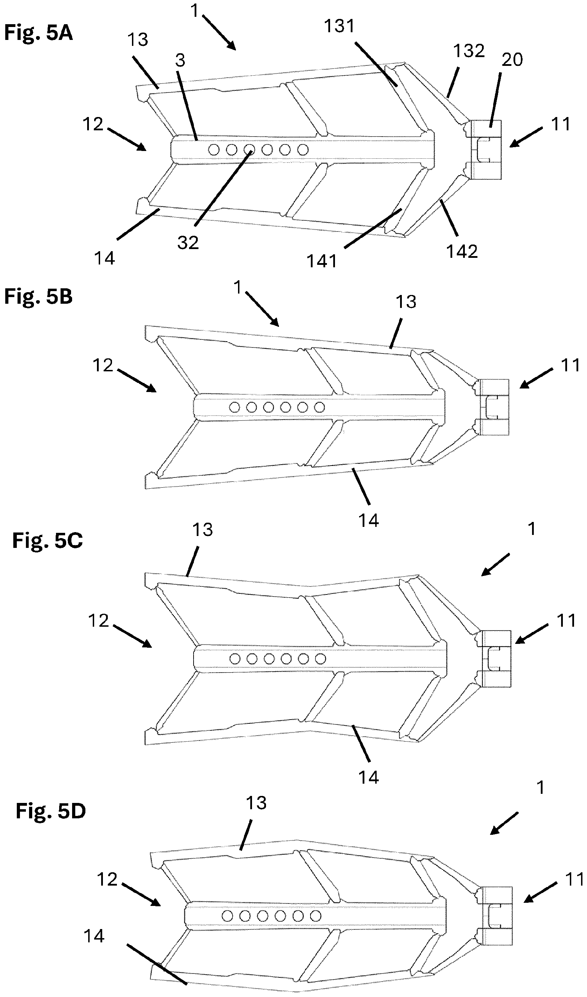

De même, dans divers modes de réalisation qui ne concernent pas nécessairement des os longs, l'implant comporte des bras support au niveau distal qui ont une longueur différente de celle des bras support au niveau proximal et/ou des bras de support central, de sorte que l'implant en configuration déployée possède des plateaux qui ne sont pas parallèles entre eux. Des exemples illustratifs et non limitatifs de tels modes de réalisation sont représentés sur les figures 4A, 4B, 4C et 4D en configurations repliées et sur les figures 5A, 5B, 5C et 5D correspondant à leurs configurations déployées respectives. On comprend que des bras plus longs au niveau proximal (Fig. 4A et 5A) permet d'obtenir des plateaux inclinés en direction de l'extrémité distale, tandis que des bras plus longs au niveau distal (Fig. 4B et 5B) permet d'obtenir des plateaux inclinés en direction de l'extrémité proximale. De plus, il est possible de prévoir des plateaux qui ne restent pas plans à la fin de l'expansion, ce qui représente un net avantage de l'implant qui peut alors épouser les formes anatomiques des os où ils sont implantés. Ainsi, une forme concave ou bi-concave (i.e., concave sur les deux faces des plateaux) de l'implant déployée peut être obtenue avec des bras centraux ((130, 140) plus courts que les bras support (131, 141) comme sur les figures 4C et 5C, tandis qu'une forme convexe (ou bi-convexe : i.e., convexe sur les deux faces des plateaux) comme sur les figures 4D et 5D.

Certains modes de réalisation prévoient l'injection d'un fluide (e.g., « ciment osseux », généralement à base d'un polymère comme le PMMA par exemple et bien connu de l'homme de métier, de sorte qu'aucun détail sur le ciment ne sera fourni ici). Ainsi, l'implant une fois positionné peut notamment être stabilisé grâce une telle injection de ciment. Cependant, comme les fuites de ciment restent un problème, il est préférable de ne pas utiliser de ciment avec l'implant décrit ici si le ciment n'est pas contenu, du moins dans les cas où la fracture est importante au point que le ciment puisse fuir dans les tissus environnants.

Le terme « solidarisé » signifie ici que deux éléments sont rendus solidaires, soit de manière permanente (ou quasi-permanente) mais également parfois qu'une liaison est réalisée pour l'actionnement d'un élément par un autre. Ainsi, un vissage ou une coopération de forme pour verrouiller provisoirement les éléments entre sont couverts par ce terme non limitatif.

Les termes bague, manchon, ou tube désignent des structures creuses telles que des anneaux, des conduits ou des tuyaux mais de façon non limitative, notamment avec des formes diverses (à l'intérieur comme à l'extérieur) bien que la forme cylindrique soit préférée. Le terme canal est en revanche de préférence utilisé ici pour désigner un passage plutôt que l'élément qui le contient et le terme ouverture désigne ici le fait qu'un élément soit ouvert et apte à être traversé en débouchant dans une autre structure ou un autre élément. Généralement les termes manchon et tubes ou conduits désignent des éléments plus longs que les bagues ou anneaux, mais leur utilisation ici n'est pas limitative non plus. D'autre part, les termes douille ou culot désignent également des structures creuses qui sont ouvertes à une extrémité mais fermées à l'autre extrémité, comme des bouchons, des fermetures, des constrictions ou étranglement et ces termes sont utilisés indifféremment sans limitation quelconque.

Le terme « charnière » est utilisé ici dans son acception fonctionnelle, sans impliquer de limitation structurelle et peut désigner en fait des charnières mécaniques, même si elles sont de préférence formées (comme illustrés sur les exemples non limitatifs des figures) par des amincissement (ou rétrécissement, retrait de matière) des éléments tels que les bras de support ou autre. Ainsi, une charnière est en fait un point ou une zone d'articulation, puisqu'il est connu dans le domaine qu'il est généralement sans danger de prévoir de tels mécanismes de pivotement pour des implants car les matériaux qui les composent sont adaptés à ce type d'articulation. On notera que des amincissements des plateaux sont envisageables aussi, notamment à côté des articulations (charnières) des bras support, pour permettre au plateau de suivre la forme morphologique souhaitée, notamment lorsque les bras support n'ont pas les mêmes longueurs entre eux.

Par ailleurs, la présente invention permet de maîtriser la forme de l'implant une fois déployé, par exemple comme illustré sur les figures 4A, 4B, 4C, 4D, 5A, 5B, 5C et 5D. Un instrument d'injection de fluide (Ac) peut être prévu pour remplir l'implant d'un fluide tel que du ciment osseux Un tel instrument peut être muni de moyens de contrôler la pression injectée (un manomètre par exemple) et de savoir quel est le volume résultant, afin de contrôler efficacement l'expansion dans les tissus osseux.

Enfin, on comprend que l'instrumentation proposée dans la présente demande dans certains modes de réalisation, en utilisant un porte-implant (ou ancillaire) relativement classique pour tenir l'implant et l'introduire dans les tissus osseux, mais également moins classique pour l'expandre dans les tissus osseux, présente également l'avantage de pouvoir réaliser toutes les étapes d'implantation et de stabilisation avec un seul instrument et en une opération continue. En effet, l'ancillaire avec un tube creux d'acheminement du ciment au travers du tube qui retient le ciment, permet de disposer d'un instrument permettant d'effectuer l'opération chirurgicale rapidement et efficacement. Après le perçage, on introduit l'implant et sans retirer l'instrument, on peut injecter du ciment et ensuite retirer l'outil avant, pendant ou même après la polymérisation du ciment (par exemple à l'aide d'un mécanisme de coupe du ciment dur lors d'une rotation de l'instrument). Le temps d l'opération chirurgicale est bien entendu nettement diminué mais également la stabilité de l'implant qui n'est lâché à aucun moment tant qu'il n'est pas stabilisé par l'injection de ciment remplissant tous les volumes libres autour, contrairement à certaines solutions de l'art antérieur.

D'une manière générale, la présente demande concerne un implant (1) osseux expansible de chirurgie orthopédique humaine pour la restauration de volume et/ou géométrie d'un os, par une expansion entre une configuration repliée et une configuration déployée, ledit implant comportant un axe central (3) et s'étendant selon un axe longitudinal (L) entre une extrémité proximale (11) apte à coopérer avec un instrument d'implantation (A) pour tenir l'implant et une extrémité distale (12) destinée à être insérée en premier dans l'os, au moins deux faces, par exemple supérieure et inférieure, de l'implant comportant chacune au moins un plateau (13, 14, 15) pour le contact avec les tissus osseux, chacun des plateaux étant supportés par au moins deux bras de support (131, 141, 151) chacun, par l'intermédiaire d'une charnière sur l'axe central (3) et d'une charnière sous le plateau (13, 14, 15) respectif de chacun desdits bras de support (131, 141, 151) ;
caractérisé en ce que :
- une douille ou bague d'expansion (20) disposée dans le même axe que ledit axe central (3) ;
- au moins deux bras d'expansion (132, 142, 152) comportent chacun une charnière les reliant à l'une des extrémités de l'un des plateaux (13, 14, 15) et une charnière les reliant à ladite douille ou bague d'expansion (20) ;
- ladite douille ou bague d'expansion (20) et l'extrémité proximale de l'axe central (3) sont aptes à coopérer, respectivement ou inversement, avec un tube creux (A1) de préhension de l'implant (1) d'un instrument d'implantation (A) et avec une tige d'expansion (A3) dudit instrument (A) ;
- ladite tige d'expansion (A3) est apte à coulisser à l'intérieur dudit tube creux (A1), de façon à permettre de provoquer un éloignement entre ladite douille (3) et ledit axe central (3), exerçant une traction sur les plateaux (13, 14, 15), par l'intermédiaire des bras d'expansion (132, 142, 152), ce qui engendre un pivotement desdits bras de support (131, 141, 151) provoquant l'éloignement des plateaux (13, 14, 15) par rapport à l'axe central (3), de manière à résulter en une expansion contrôlée de l'implant (1) entre ladite configuration repliée et ladite configuration déployée.

Dans certains modes de réalisation, c'est ladite bague d'expansion (20) qui est apte à coopérer avec un tube creux (A1) de préhension de l'implant (1) dudit instrument d'implantation (A), en laissant passer la tige d'expansion (A3) à travers la bague, tandis que l'axe central (3) est apte à coopérer avec ladite tige d'expansion (A3) dudit instrument (A), de sorte qu'une poussée exercée sur ladite tige d'expansion (A3) coulissant à l'intérieur dudit tube creux (A1) provoque l'éloignement dudit axe central (3) par rapport à ladite bague (20), résultant en une expansion de l'implant (1) contrôlée en fonction de la force de poussée exercée sur la tige d'expansion (A3).

Dans certains modes de réalisation alternatifs des précédents, c'est l'extrémité proximale de l'axe central (3) qui est apte à coopérer avec ledit tube creux (A1) de préhension de l'implant (1) dudit instrument d'implantation (A), tandis que ladite douille ou bague d'expansion (20) est apte à coopérer avec ladite tige d'expansion (A3) dudit instrument (A), de sorte qu'une poussée exercée sur ladite tige d'expansion (A3) coulissant à l'intérieur dudit tube creux (A1) provoque l'éloignement de ladite douille ou bague d'expansion (20) par rapport audit axe central (3), résultant en une expansion de l'implant (1) contrôlée en fonction de la force de poussée exercée sur la tige d'expansion (A3).

Dans certains modes de réalisation, l'axe central (3) comporte un conduit (31) apte à coopérer avec ladite tige d'expansion (A3) qui est, d'une part, creuse et munie d'au moins une ouverture au niveau de son extrémité distale et, d'autre part, connectable à un instrument (AC) d'injection de fluide pour acheminer au moins un fluide dans le site d'implantation de l'implant via l'axe central (3), de préférence muni d'ouvertures (32) pour faire déboucher le fluide dans la longueur de l'axe central (3).

Dans certains modes de réalisation, les bras de support (131, 141, 151) des plateaux comportent deux bras reliés respectivement à proximité des extrémités proximale et distale de leur plateau (13, 14, 15) respectif, pour fournir un support sur toute la longueur des plateaux et limiter leurs risques de cintrage ou fluage.

Implant selon l'une des revendications précédentes, caractérisé en ce qu'au moins un bras de support central (130, 140, 150) supplémentaire est relié entre une portion centrale de l'axe central et une portion centrale des plateaux (13, 14, 15), avec des charnières aux deux extrémités du bras de support (130, 140, 150) pour son pivotement.

Dans certains modes de réalisation, deux plateaux (13, 14) disposés de part et d'autre de l'axe central (3) pour fournir un appui contre des tissus osseux lésés de part et d'autre de l'implant, par exemple pour restaurer une hauteur ou une largeur.

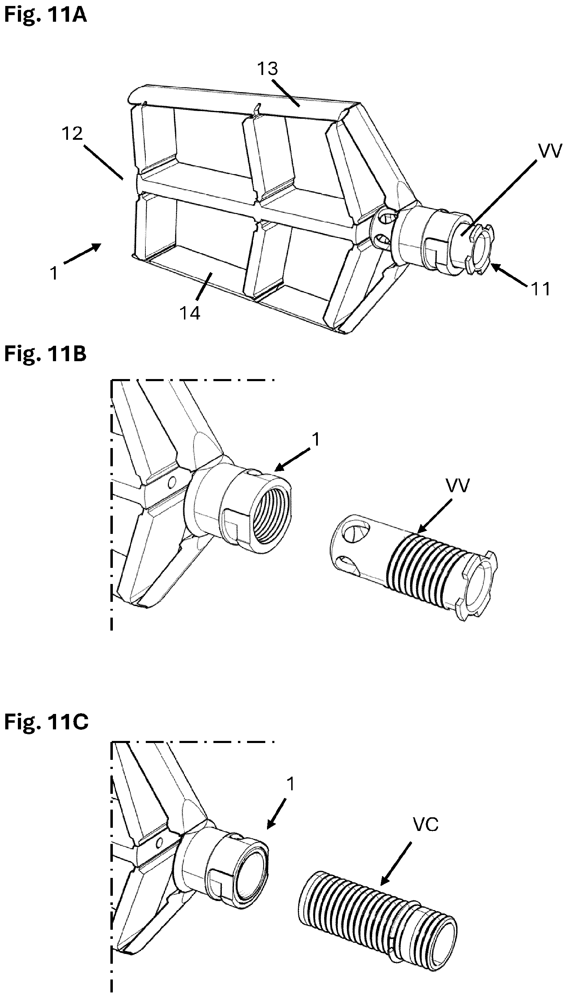

Dans certains modes de réalisation, l'implant comporte au moins un plateau (15) additionnel, la répartition des plateaux autour de l'axe central (3) variant en fonction de leur nombre et/ou des besoins en termes de traitement chirurgical, avec de préférence une équi-répartition angulaire radialement par rapport à l'axe central, pour exercer une compression homogène sur les tissus osseux à la périphérie de l'implant. Dans certains modes de réalisation, des bras support en doubles exemplaires sont prévus pour renforcer la structure. D'autre part, des moyens de verrouillage sont prévus dans certains cas pour empêcher l'implant de se replier. Les diamètres très fins des implants et leurs axes centraux sont difficilement compatibles avec des filetages pour opérer l'expansion par vissage au niveau de l'implant, alors qu'il est avantageux d'effectuer un vissage au niveau de l'instrument actionnant l'expansion, notamment lorsque l'expansion implique un rapprochement des bras support les uns par rapport aux autres. Ainsi, comme connu de l'art antérieur, il est possible d'utiliser par exemple une bague fendue logée dans un renforcement circulaire de l'implant et coopérant avec des crans sur l'axe de poussée ou de traction qui sont orientés pour permettre le passage de cet axe dans un seul sens, par exemple comme représenté sur les figures 11C. Ainsi, l'actionnement de l'axe pour l'expansion des plateaux peut être réalisé par passage successifs des crans, ce qui permet de verrouiller l'implant en configuration déployée.

Cependant, contrairement à certains implants de l'art antérieur où l'axe de traction permettant de rapprocher doit nécessairement rester en place, les implants de la présente demande sont déployés sans rapprochement des bras de support, ce qui permet avantageusement de pouvoir les verrouiller avec un verrou vissé et de ne pas nécessiter de tels crans rendant la tâche difficile et offrant une fiabilité réduite. Ainsi, par exemple comme représenté sur les figures 11A et 11B, il est possible d'utiliser par exemple un manchon fileté configuré pour être disposé dans le tube creux de l'instrument d'implantation (A) tenant l'implant (et entourer l'éventuel conduit d'injection de fluide présent à l'intérieur). Un tel manchon possède alors un filetage prévu pour coopérer avec un taraudage de l'extrémité proximale (11) de l'implant et présente des moyens d'actionnement pour le vissage ou dévissage (comme des ailettes radiales représentées sur la figure 11C).

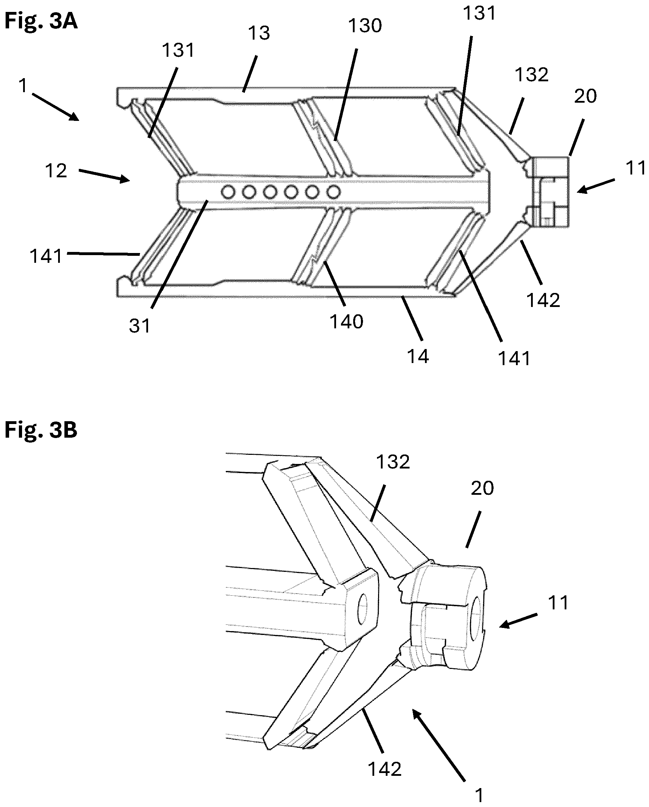

De plus, l'agencement des implants de la présente demande fournit un avantage non négligeable en ce qui concerne la fiabilité de l'expansion. En effet, par le fait que les bras support sont disposés au moins aux extrémités des plateaux (et éventuellement avec un ou plusieurs bras de renfort entre les extrémités) permet de créer des parallélogrammes déformables qui conservent leur propriété de parallélisme entre leurs côtés, contrairement à certains implants de l'art antérieur où les bras de support sont montés en opposition. Ce type d'implants de l'art antérieur nécessitent généralement que les bras soient prévus en doubles exemplaires de chaque côté pour améliorer la fiabilité de l'expansion. Les implants de la présente demande ne nécessitent pas de doubler les bras, mais il reste cependant possible de le faire, notamment dans le cas d'implants de grande taille et/ou destinés à supporter une charge importante. Ainsi, certains modes de réalisation comportent des bras supports en double exemplaire, au moins à l'une des positions de ces bras et de préférence à chacun d'entre elles, par exemple comme représenté sur la figure 3A. De plus, de tels bras doubles peuvent comporter un mécanisme d'auto-verrouillage en configuration déployée, comme par exemples des crans ménagés face à face de sorte à s'engager entre eux, par exemple comme représenté pour les bras de support central (130, 140) de la figure 3A.

La présente demande concerne aussi un système de traitement orthopédique de tissu osseux lésé comprenant un ciment de substitution osseuse et au moins un instrument d'implantation (A) et d'injection de ciment (Ac) dans l'implant (1), caractérisé en ce qu'il comporte un implant (1) selon divers modes de réalisation.

Dans certains modes de réalisation, l'instrument d'implantation (A) et d'injection de ciment (Ac) comporte des moyens de contrôle de la pression et/ou de l'aspiration du ciment pour replier l'implant en configuration repliée si nécessaire.

Dans certains modes de réalisation, l'instrument d'implantation (A) est distinct mais complémentaire de l'instrument d'injection (Ac) dont le canal d'injection du ciment traverse un canal à l'intérieur de la tige de l'instrument d'implantation (A) tenant l'extrémité proximale de l'implant (1) grâce à son extrémité distale.

La présente demande décrit diverses caractéristiques techniques et avantages en référence aux figures et/ou à divers modes de réalisation. L'homme de métier comprendra que les caractéristiques techniques d'un mode de réalisation donné peuvent en fait être combinées avec des caractéristiques d'un autre mode de réalisation à moins que l'inverse ne soit explicitement mentionné ou qu'il ne soit évident que ces caractéristiques sont incompatibles ou que la combinaison ne fournisse pas une solution à au moins un des problèmes techniques mentionnés dans la présente demande. De plus, les caractéristiques techniques décrites dans un mode de réalisation donné peuvent être isolées des autres caractéristiques de ce mode à moins que l'inverse ne soit explicitement mentionné.

### Liste détaillée des références dans les figures :

- 1: implant
- 11: extrémité proximale
- 12: extrémité distale
- A: instrument d'implantation
- Ac: instrument d'injection de fluide
- A1: tube creux de préhension
- A3: tige d'expansion ?
- 3: axe central
- 31: conduit dans l'axe central
- 32: ouvertures du conduit de l'axe central
- 13: premier plateau
- 14: deuxième plateau
- 15: troisième plateau
- 20: bague d'expansion
- 131: bras support du premier plateau
- 141: bras support du deuxième plateau
- 151: bras support du troisième plateau
- 132: bras d'expansion du premier plateau
- 142: bras d'expansion du deuxième plateau
- 152: bras d'expansion du troisième plateau
- 130: bras support central du premier plateau
- 140: bras support central du deuxième plateau
- 150: bras support central du troisième plateau
- VC: verrou cranté
- VV: verrou vissé

## Revendications

1. Implant (1) osseux expansible de chirurgie orthopédique humaine pour la restauration de volume et/ou géométrie d'un os, par une expansion entre une configuration repliée et une configuration déployée, ledit implant comportant un axe central (3) et s'étendant selon un axe longitudinal (L) entre une extrémité proximale (11) apte à coopérer avec un instrument d'implantation (A) pour tenir l'implant et une extrémité distale (12) destinée à être insérée en premier dans l'os, au moins deux faces, par exemple supérieure et inférieure, de l'implant comportant chacune au moins un plateau (13, 14, 15) pour le contact avec les tissus osseux, chacun des plateaux étant supportés par au moins deux bras de support (131, 141, 151) chacun, par l'intermédiaire d'une charnière sur l'axe central (3) et d'une charnière sous le plateau (13, 14, 15) respectif de chacun desdits bras de support (131, 141, 151) ;
**caractérisé en ce que** :
- une douille ou bague d'expansion (20) disposée dans le même axe que ledit axe central (3) ;
- au moins deux bras d'expansion (132, 142, 152) comportent chacun une charnière les reliant à l'une des extrémités de l'un des plateaux (13, 14, 15) et une charnière les reliant à ladite douille ou bague d'expansion (20) ;
- ladite douille ou bague d'expansion (20) et l'extrémité proximale de l'axe central (3) sont aptes à coopérer, respectivement ou inversement, avec un tube creux (A1) de préhension de l'implant (1) d'un instrument d'implantation (A) et avec une tige d'expansion (A3) dudit instrument (A) ;
- ladite tige d'expansion (A3) est apte à coulisser à l'intérieur dudit tube creux (A1), de façon à permettre de provoquer un éloignement entre ladite douille (3) et ledit axe central (3), exerçant une traction sur les plateaux (13, 14, 15), par l'intermédiaire des bras d'expansion (132, 142, 152), ce qui engendre un pivotement desdits bras de support (131, 141, 151) provoquant l'éloignement des plateaux (13, 14, 15) par rapport à l'axe central (3), de manière à résulter en une expansion contrôlée de l'implant (1) entre ladite configuration repliée et ladite configuration déployée.

2. Implant selon la revendication 1, **caractérisé en ce que** c'est ladite bague d'expansion (20) qui est apte à coopérer avec un tube creux (A1) de préhension de l'implant (1) dudit instrument d'implantation (A), en laissant passer la tige d'expansion (A3) à travers la bague, tandis que l'axe central (3) est apte à coopérer avec ladite tige d'expansion (A3) dudit instrument (A), de sorte qu'une poussée exercée sur ladite tige d'expansion (A3) coulissant à l'intérieur dudit tube creux (A1) provoque l'éloignement dudit axe central (3) par rapport à ladite bague (20), résultant en une expansion de l'implant (1) contrôlée en fonction de la force de poussée exercée sur la tige d'expansion (A3).

3. Implant selon la revendication 1, **caractérisé en ce que** c'est l'extrémité proximale de l'axe central (3) qui est apte à coopérer avec ledit tube creux (A1) de préhension de l'implant (1) dudit instrument d'implantation (A), tandis que ladite douille ou bague d'expansion (20) est apte à coopérer avec ladite tige d'expansion (A3) dudit instrument (A), de sorte qu'une poussée exercée sur ladite tige d'expansion (A3) coulissant à l'intérieur dudit tube creux (A1) provoque l'éloignement de ladite douille ou bague d'expansion (20) par rapport audit axe central (3), résultant en une expansion de l'implant (1) contrôlée en fonction de la force de poussée exercée sur la tige d'expansion (A3).

4. Implant selon l'une des revendications précédentes, **caractérisé en ce que** les plateaux sont munis, sur leur surface de contact avec le tissu osseux, d'irrégularités de forme tels que des protubérances ou à l'inverse invaginations pour améliorer l'accroche des plateaux sur le tissu osseux (DESC : crans nervures, rainures, pointes chevrons etc)

5. Implant selon l'une des revendications précédentes, **caractérisé en ce que** l'axe central (3) comporte un conduit (31) apte à coopérer avec ladite tige d'expansion (A3) qui est, d'une part, creuse et munie d'au moins une ouverture au niveau de son extrémité distale et, d'autre part, connectable à un instrument (AC) d'injection de fluide pour acheminer au moins un fluide dans le site d'implantation de l'implant via l'axe central (3), de préférence muni d'ouvertures (32) pour faire déboucher le fluide dans la longueur de l'axe central (3).

6. Implant selon l'une des revendications précédentes, **caractérisé en ce que** les bras de support (131, 141, 151) des plateaux comportent deux bras reliés respectivement à proximité des extrémités proximale et distale de leur plateau (13, 14, 15) respectif, pour fournir un support sur toute la longueur des plateaux et limiter leurs risques de cintrage ou fluage.

7. Implant selon l'une des revendications précédentes, **caractérisé en ce qu'**au moins un bras de support central (130, 140, 150) supplémentaire est relié entre une portion centrale de l'axe central et une portion centrale des plateaux (13, 14, 15), avec des charnières aux deux extrémités du bras de support (130, 140, 150) pour son pivotement.

8. Implant selon l'une des revendications précédentes, **caractérisé en ce que** les bras support au niveau distal ont une longueur différente de celle des bras support au niveau proximal et des bras de support centraux.

9. Implant selon l'une des revendications précédentes, **caractérisé en ce qu'**il comporte deux plateaux (13, 14) disposés de part et d'autre de l'axe central (3) pour fournir un appui contre des tissus osseux lésés de part et d'autre de l'implant, par exemple pour restaurer une hauteur ou une largeur.

10. Implant selon l'une des revendications 1 à 6, **caractérisé en ce qu'**il comporte au moins un plateau (15) additionnel, la répartition des plateaux autour de l'axe central (3) variant en fonction de leur nombre et/ou des besoins en termes de traitement chirurgical, avec de préférence une équi-répartition angulaire radialement par rapport à l'axe central, pour exercer une compression homogène sur les tissus osseux à la périphérie de l'implant.

11. Système de traitement orthopédique de tissu osseux lésé comprenant un ciment de substitution osseuse et au moins un instrument d'implantation (A) et d'injection de ciment (Ac) dans l'implant (1), **caractérisé en ce qu'**il comporte un implant (1) selon l'une des revendications précédentes.

12. Système selon la revendication 11, **caractérisé en ce que** l'instrument d'implantation (A) et d'injection de ciment (Ac) comporte des moyens de contrôle de la pression et/ou de l'aspiration du ciment pour replier l'implant en configuration repliée si nécessaire.

13. Système selon l'une des revendications 11 et 12, **caractérisé en ce que** l'instrument d'implantation (A) est distinct mais complémentaire de l'instrument d'injection (Ac) dont le canal d'injection du ciment traverse un canal à l'intérieur de la tige de l'instrument d'implantation (A) tenant l'extrémité proximale de l'implant (1) grâce à son extrémité distale.
